# EUROPÄISCHE PATENTSCHRIFT

(11) **EP 4 069 107 B1**
(45) Veröffentlichungstag und Bekanntmachung des Hinweises auf die Patenterteilung: **28.01.2026**
(21) Anmeldenummer: 20817179.3
(22) Anmeldetag: 03.12.2020
(51) Int. Cl.: A61B 17/34, A61B 34/10, A61B 90/11, A61B 90/00

(54) **NADELFÜHRUNGSVORRICHTUNG UND SET MIT MEHREREN ELEMENTEN**
NEEDLE GUIDING DEVICE, AND SET COMPRISING A PLURALITY OF ELEMENTS
DISPOSITIF DE GUIDAGE D'AIGUILLE ET ENSEMBLE COMPRENANT UNE PLURALITÉ D'ÉLÉMENTS

(30) Priorität: 06.12.2019 DE 102019133421
(43) Veröffentlichungstag der Anmeldung: 12.10.2022
(73) Patentinhaber: InLine-Med GmbH, 39106 Magdeburg (DE)
(72) Erfinder: SÁNCHEZ LÓPEZ, Juan Sebastián, 39124 Magdeburg (DE); LAGOTZKI, Sinja, 39104 Magdeburg (DE)
(74) Vertreter: Leach, Sean Adam
(86) Internationale Anmeldenummer: PCT/EP2020/084477
(87) Internationale Veröffentlichungsnummer: WO 2021/110840

(56) Entgegenhaltungen:
- EP-A2- 1 958 588
- DE-A1- 10 029 737
- DE-B4- 10 029 737
- US-A- 4 883 053
- US-A1- 2009 177 077
- US-A1- 2018 228 568
- US-B2- 9 125 676

## Beschreibung

Die Erfindung betrifft Nadelführungsvorrichtung zur Führung und Positionierung einer nadelförmigen Vorrichtung an einem Patienten, wobei die Nadelführungsvorrichtung ein Basiselement und ein Nadelführungselement aufweist, an dem die nadelförmige Vorrichtung entlang ihrer Längserstreckung führbar ist, wobei die Winkelorientierung des Nadelführungselements gegenüber dem Basiselement in zwei voneinander unabhängigen Winkelfreiheitsgraden einstellbar ist. Die Erfindung betrifft außerdem ein Set mit mehreren Elementen für die Durchführung einer Untersuchung und/oder Behandlung eines Patienten.

Allgemein betrifft die Erfindung das Gebiet medizinischer Vorrichtungen, die für die Untersuchung und/oder Behandlung von Patienten eingesetzt werden können, beispielsweise im Rahmen einer Biopsie oder Schmerztherapie. Diese Prozeduren werden in der Regel manuell unter Bildgebung durch Ultraschalt, Computertomographie oder Magnetresonanztomographie durchgeführt. Es ist bereits bekannt, zur Unterstützung des Arztes eine mechanische Hilfsvorrichtung in Form einer Nadelführungsvorrichtung einzusetzen, die die Ausrichtung der Biopsienadel vereinfacht und während der Durchführung der Biopsie beibehält. Eine derartige Nadelführungsvorrichtung ist zum Beispiel aus der WO 2019/101862 A1 bekannt.

Aus der US 9,125,676 B2 ist eine verstellbare Nadelführungsvorrichtung für eine bildgeführte Intervention am Patienten bekannt. Aus der US 4,883,053 ist ein selbststabilisierendes Angulierungsgerät für eine präzise perkutane Insertion einer Nadel oder einem ähnlichen Objekt bekannt. Aus der DE 100 29 737 A1 ist eine Vorrichtung für eine Navigation eines medizinischen Instruments bekannt. Aus der US 2018/0228568 A1 ist eine medizinische Führungsvorrichtung bekannt. Aus der US 2009/0177077 A1 sind MRT-kompatible Patches und Verfahren zu deren Nutzung bekannt. Aus der EP 1 958 588 A2 ist eine medizinische Führung zum Führen eines medizinischen Instruments bekannt.

Der Erfindung liegt die Aufgabe zugrunde, weiter verbesserte Hilfsmittel für eine Untersuchung und/oder Behandlung eines Patienten unter Nutzung einer nadelförmigen Vorrichtung anzugeben.

Diese Aufgabe wird mit den Merkmalen des Anspruchs 1 gelöst. Dies umfasst eine Nadelführungsvorrichtung zur Führung und Positionierung einer nadelförmigen Vorrichtung an einem Patienten, wobei die Nadelführungsvorrichtung ein Basiselement und ein Nadelführungselement aufweist, an dem die nadelförmige Vorrichtung entlang ihrer Längserstreckung führbar ist, wobei die Winkelorientierung des Nadelführungselements gegenüber dem Basiselement in zwei voneinander unabhängigen Winkelfreiheitsgraden einstellbar ist, mit folgenden Merkmalen:
a) die Nadelführungsvorrichtung weist ein Bedienelement auf, durch das die Winkelorientierung des Nadelführungselements in den zwei Winkelfreiheitsgraden fixierbar oder eine solche Fixierung wieder lösbar ist,
b) die Nadelführungsvorrichtung weist für jeden der zwei einstellbaren Winkelfreiheitsgrade eine Winkelskala und einen der jeweiligen Winkelskala zugeordneten Winkelzeiger auf, sodass durch den Anwender eine aktuell eingestellte Winkelorientierung des Nadelführungselements in den zwei Winkelfreiheitsgraden anhand der Position des jeweiligen Winkelzeiger gegenüber der zugeordneten Winkelskala ablesbar ist.

Die erfindungsgemäße Nadelführungsvorrichtung führt zu einer deutlichen Vereinfachung der Durchführung einer Untersuchung und/oder Behandlung eines Patienten mittels einer nadelförmigen Vorrichtung, beispielsweise bei einer Biopsie. So kann durch ein einziges Bedienelement die Winkelorientierung des Nadelführungselements in zwei Winkelfreiheitsgraden fixiert werden oder wieder gelöst werden. Auf diese Weise eignet sich die Nadelführungsvorrichtung für eine Einhandbedienung, zumindest soweit es um die Betätigung des Bedienelements geht. Zudem wird die Benutzung der Nadelführungsvorrichtung durch das Vorhandensein jeweiliger Winkelskalen und zugeordneter Winkelzeiger für jeden der zwei einstellbaren Winkelfreiheitsgrade vereinfacht. Der Anwender kann auf diese Weise schnell und ohne zusätzliches Zubehör die erforderlichen Winkeleinstellungen anhand der Betrachtung der Winkelzeiger und Winkelskalen durchführen.

Die Nadelführungsvorrichtung kann vorteilhafterweise derart ausgebildet sein, dass bei jeder Einstellung der Winkelorientierung des Nadelführungselements in den zwei Winkelfreiheitsgraden eine durch das Nadelführungselement geführte nadelförmige Vorrichtung immer an derselben Position in Kontakt mit dem Körper des Patienten kommt, das heißt bei einer Biopsie trifft die Biopsienadel immer dieselbe Einstichstelle. Das Basiselement dient dabei zur Anbringung der Nadelführungsvorrichtung am Körper des Patienten, beispielsweise mittels eines wieder lösbaren Klebstoffs.

Die nadelförmige Vorrichtung kann beispielsweise eine Biopsienadel, eine Injektionsnadel oder eine Ablationsnadel sein.

Gemäß einer vorteilhaften Ausgestaltung der Erfindung ist vorgesehen, dass die Nadelführungsvorrichtung einen durch das Bedienelement betätigbaren Klemmmechanismus aufweist, wobei die Winkelorientierung des Nadelführungselements gegenüber dem Basiselement in den zwei Winkelfreiheitsgraden durch Bedienung des Klemmmechanismus mittels des Bedienelements durch Klemmung fixierbar ist. Ein solcher Klemmmechanismus kann auf vergleichsweise einfache Art realisiert werden, beispielsweise mit gegeneinander anziehbaren Gewindeelementen, durch die ein Teil der Nadelführungsvorrichtung gegeneinander verspannt wird und auf diese Weise die Fixierung der Winkelorientierung des Nadelführungselements durch Klemmung ermöglicht. Mit einem Klemmmechanismus kann insbesondere eine stufenlose Verstellung der Winkelorientierung des Nadelführungselements gegenüber dem Basiselement in den zwei Winkelfreiheitsgraden realisiert werden. Auf diese Weise kann eine Rastung vermieden werden, sodass grundsätzlich beliebige Winkelorientierungen im möglichen Verschwenkbereich des Nadelführungselements eingestellt werden. Die Einstellung der Winkelorientierung des Nadelführungselements kann in zumindest einem Winkelfreiheitsgrad oder beiden Winkelfreiheitsgraden auch stufig erfolgen, beispielsweise über Rastelemente.

Gemäß einer vorteilhaften Ausgestaltung der Erfindung ist vorgesehen, dass die Winkelorientierung des Nadelführungselements gegenüber dem Basiselement in den zwei Winkelfreiheitsgraden stufenlos einstellbar ist. Auf diese Weise ist eine praktisch beliebige Einstellung der Winkelorientierung in beiden Winkelfreiheitsgraden möglich, sodass der Anwender durch die Konstruktion der Nadelführungsvorrichtung hinsichtlich seiner Einstellmöglichkeiten nicht limitiert ist.

Gemäß der Erfindung ist vorgesehen, dass die Nadelführungsvorrichtung einen Haltebügel aufweist, an dem das Nadelführungselement befestigt ist, wobei der Haltebügel in einem ersten der zwei Winkelfreiheitsgrade gegenüber dem Basiselement bewegbar ist und das Nadelführungselement in einem zweiten der zwei Winkelfreiheitsgrade gegenüber dem Haltebügel bewegbar ist. Dies erlaubt eine einfache und robuste Konstruktion der Nadelführungsvorrichtung. Der Haltebügel kann z.B. bogenförmig ausgebildet sein und durch seine Bogenform eine bogenförmige Bewegbarkeit des Nadelführungselements gegenüber dem Haltebügel unterstützten. Das Nadelführungselement kann zum Beispiel in der Art eines Schlittens oder mittels eines Halteschlittens an dem Haltebügel geführt sein und in einer bogenförmigen Bewegung, die der Bogenform des Haltebügels entspricht, entlang des Haltebügels verschiebbar und auf diese Weise gegenüber dem Haltebügel bewegbar sein. Der Haltebügel kann beispielsweise um eine Schwenkachse verschwenkbar an dem Basiselement befestigt sein.

Gemäß der Erfindung ist vorgesehen, dass die Nadelführungsvorrichtung einen Klemmbügel aufweist, der an dem Basiselement befestigt ist und um dieselbe Schwenkachse gegenüber dem Basiselement verschwenkbar ist wie der Haltebügel. Der Klemmbügel kann dabei einen Teil des erwähnten Klemmmechanismus bilden. Beispielsweise kann der Klemmbügel mit dem Haltebügel gekoppelt sein, sodass der Klemmbügel bei einer Bewegung des Haltebügels gegenüber dem Basiselement die gleiche Bewegung, zum Beispiel die erwähnte Verschwenkbewegung, mit ausführt. Der Klemmbügel kann zum Beispiel zwischen dem Haltebügel und dem Basiselement angeordnet sein. Der Klemmbügel kann z.B. bogenförmig ausgebildet sein, z.B. mit einer Bogenform, die konzentrisch zur Bogenform des Haltebügels verläuft.

Der Haltebügel und der Klemmbügel können beispielsweise zumindest im Wesentlichen parallel zueinander verlaufen, d. h. zwei im Wesentlichen parallel zueinander verlaufende Bögen bilden. Dabei kann zwischen dem Haltebügel und dem Klemmbügel ein gewisser Abstand oder Freiraum vorhanden sein. Durch die Bogenform zumindest des Klemmbügels weist die Nadelführungsvorrichtung in dem Bereich zwischen dem Klemmbügel und den Basiselement einen Freiraum auf, der Beispiel im Wesentlichen ein kreisförmig oder, bezogen auf die Verschwenkmöglichkeit des Klemmbügels, halbkugelförmig ist. Der Klemmbügel kann mit den Haltebügel hinsichtlich der Verschwenkbarkeit gekoppelt sein, sodass der Klemmbügel über immer um den gleichen Winkel verschwenkt wird wie der Haltebügel.

Beim Fixieren des Nadelführungselements in den zwei Winkelfreiheitsgraden mittels des Bedienelements können der Haltebügel und der Klemmbügel gegeneinander verspannt werden, wofür der Freiraum zwischen dem Haltebügel und dem Klemmbügel genutzt werden kann. Dabei können Haltebügel und/oder der Klemmbügel etwas verformt werden. Die Klemmung am Basiselement kann z.B. an bogenförmigen Bereichen des Basiselements erfolgen, an denen der Haltebügel und/oder der Klemmbügel verschwenkbar gelagert sind. Auf diese Weise kann durch Betätigung des einen Bedienelements eine Art "Fernklemmung" erfolgen, d. h. es wird auch eine Klemmwirkung an einer vom Bedienelement entfernten Stelle ausgelöst.

Wie erwähnt, kann die Nadelführungsvorrichtung derart ausgebildet sein, dass bei jeder Einstellung der Winkelorientierung des Nadelführungselements in den zwei Winkelfreiheitsgraden eine durch das Nadelführungselement geführte nadelförmige Vorrichtung immer an derselben Position in Kontakt mit dem Körper des Patienten kommt, das heißt bei einer Biopsie trifft die Biopsienadel immer dieselbe Einstichstelle. Das Basiselement kann an einer dem Patienten zugewandten Seite eine Befestigungsoberfläche haben, mit der das Basiselement am Patienten zu befestigen ist. Hierzu kann die Nadelführungsvorrichtung derart ausgebildet sein, dass sowohl die Verschwenkachse des Haltebügels (X-Achse) bezüglich des Basiselements als auch die durch die Bogenform des Haltebügels definierte Verschwenkachse des Nadelführungselements (Y-Achse) gegenüber dem Basiselement exakt in der Ebene der Befestigungsoberfläche verlaufen.

Gemäß einer vorteilhaften Ausgestaltung der Erfindung ist vorgesehen, dass eine erste Winkelskala an dem Haltebügel und/oder dem Klemmbügel angeordnet ist und/oder eine zweite Winkelskala im Bereich einer Befestigungsanordnung des Haltebügels an dem Basiselement angeordnet ist. Dies hat den Vorteil, dass vorhandene, relativ große Oberflächen dieser Bauteile Haltebügel, Klemmbügel und Basiselement für die Beschriftung durch eine jeweilige Winkelskala genutzt werden können. Auf diese Weise ist die Winkelskala gut ablesbar. Der der ersten Winkelskala zugeordnete erste Winkelzeiger kann zum Beispiel an dem Nadelführungselement oder einem mit dem Nadelführungselement verbundenen Teil, zum Beispiel dem zuvor erwähnten Halteschlitten, angeordnet sein. Der der zweiten Winkelskala zugeordnete zweite Winkelzeiger kann zum Beispiel an dem Haltebügel und/oder dem Klemmbügel angeordnet sein.

Es ist auch vorteilhaft möglich, die zuvor erläuterte Zuordnung der ersten Winkelskala zu dem Haltebügel und/oder dem Klemmbügel mit den zugeordneten Winkelzeiger zu vertauschen. Mit anderen Worten, es kann auch der erste Winkelzeiger an dem Haltebügel und/oder dem Klemmbügel angeordnet sein. In diesem Fall kann beispielsweise die dem ersten Winkelzeiger zugeordnete erste Winkelskala an dem Nadelführungselement oder einem mit dem Nadelführungselement gekoppelten Bauteil angeordnet sein. In entsprechender Weise gilt dies auch für die zweite Winkelskala. So kann alternativ der zweite Winkelzeiger im Bereich einer Befestigungsanordnung des Haltebügels an dem Basiselement angeordnet sein. In diesem Fall kann beispielsweise die dem zweiten Winkelzeiger zugeordnete zweite Winkelskala an dem Haltebügel und/oder dem Klemmbügel angeordnet sein.

Gemäß einer vorteilhaften Ausgestaltung der Erfindung ist vorgesehen, dass die Nadelführungsvorrichtung eine Nadelführungselementaufnahme aufweist, die zur Aufnahme unterschiedlich ausgebildeter Nadelführungselemente der Nadelführungsvorrichtung ausgebildet sind. Dies hat den Vorteil, dass die Nadelführungsvorrichtung sehr universell für unterschiedlichste Untersuchungen und/oder Behandlungen an Patienten verwendet werden kann. So kann die Nadelführungsvorrichtung durch Austausch eines Nadelführungselements beispielsweise an nadelförmige Vorrichtungen mit unterschiedlichen Durchmessern angepasst werden.

Gemäß einer vorteilhaften Ausgestaltung der Erfindung ist vorgesehen, dass sich die Nadelführungselementaufnahme von dem Haltebügel zu dem Klemmbügel hin erstreckt. Auf diese Weise kann der Haltebügel über die Nadelführungselementaufnahme mit dem Klemmbügel gekoppelt sein. Dementsprechend kann die gesamte Nadelführungsvorrichtung mit sehr wenigen Bauteilen realisiert sein, da die Nadelführungselementaufnahme mehrere Funktionen erfüllen kann, nämlich die Aufnahme unterschiedlich ausgebildeter Nadelführungselemente sowie die Kopplung zwischen dem Haltebügel und dem Klemmbügel. Zudem kann an der Nadelführungselementaufnahme ein weiterer Teil des Klemmmechanismus angeordnet sein, beispielsweise ein Gewinde.

Gemäß einer vorteilhaften Ausgestaltung der Erfindung ist vorgesehen, dass das Bedienelement als ringförmiges Bedienelement mit einem inneren Hohlraum ausgebildet ist, wobei sich zumindest ein Teil des Nadelführungselements durch den inneren Hohlraum hindurch erstreckt. Das Bedienelement kann zum Beispiel in der Art einer Mutter mit einem Innengewinde ausgebildet sein. Das Innengewinde kann ein zu dem Gewinde der Nadelführungselementaufnahme passendes Gewinde sein, sodass das Bedienelement nach Art einer Mutter mit der Nadelführungselementaufnahme verschraubt werden kann. Durch diese Gewindeverbindung zwischen dem Bedienelement und der Nadelführungselementaufnahme kann der Klemmmechanismus aktiviert werden, beispielsweise indem durch Anziehen der Gewindeverbindung die Klemmung zur Fixierung des Nadelführungselements gegenüber dem Basiselement in den zwei Winkelfreiheitsgraden erfolgt. Zudem kann hierdurch ein besonders kompakter Aufbau der Nadelführungsvorrichtung realisiert werden.

Gemäß einer vorteilhaften Ausgestaltung der Erfindung ist vorgesehen, dass das Basiselement eine Auflagefläche zur Auflage an dem Körper des Patienten aufweist. Die Auflagefläche kann z.B. eine schmale längliche Rechteckform, ggf. mit abgerundeten Ecken, aufweisen. Auf diese Weise benötigt die Nadelführungsvorrichtung nur wenig Platz für die Auflagefläche an dem Körper des Patienten, sodass sie sehr universell einsetzbar ist, zum Beispiel auch bei kleinen Patienten. Die Auflagefläche kann als ebene Fläche ausgebildet sein.

Die eingangs genannte Aufgabe wird außerdem gelöst durch ein Set gemäß Anspruch 6 mit mehreren

Elementen für die Durchführung einer Untersuchung und/oder Behandlung an einem Körper eines Patienten, wobei das Set wenigstens folgende Elemente aufweist:
a) eine erfindungsgemäße Nadelführungsvorrichtung zur Führung und Positionierung einer nadelförmigen Vorrichtung an dem Patienten, wobei die Nadelführungsvorrichtung ein Basiselement und ein Nadelführungselement aufweist, an dem die nadelförmige Vorrichtung entlang ihrer Längserstreckung führbar ist, wobei die Winkelorientierung des Nadelführungselements gegenüber dem Basiselement in zwei voneinander unabhängigen Winkelfreiheitsgraden einstellbar ist, wobei das Basiselement einen Grundkörper aufweist, der eine Nadeldurchführöffnung aufweist und wobei der Grundkörper mehrere Positionsmarkierungen aufweist, wobei mittels dieser Positionsmarkierungen eine präzise Positionierung der Nadelführungsvorrichtung entsprechend einem patientenbezogenen Koordinatensystem am Patienten erfolgen kann,
b) einen Rechner mit einem Computerprogramm oder zumindest ein solches Computerprogramm für einen Rechner, wobei das Computerprogramm dazu eingerichtet ist, anhand von
   - Kenndaten der Nadelführungsvorrichtung,
   - Untersuchungsdaten des Patienten aus einer bildgebenden Untersuchung des Patienten und
   - wenigstens eines vorgegebenen, mit der Nadelführungsvorrichtung durchzuführenden Untersuchungs- und/oder Behandlungsschritts
      b1) Positionierungsangaben zur Positionierung der Nadelführungsvorrichtung an dem Patienten
         und
      b2) eine oder mehrere Winkelorientierungsangaben zur Einstellung der Winkelorientierung des Nadelführungselements gegenüber dem Basiselement in dem wenigstens einen Winkelfreiheitsgrad oder den mehreren voneinander unabhängigen Winkelfreiheitsgraden
         zu berechnen und dem Anwender auszugeben, wenn das Computerprogramm auf dem Rechner ausgeführt wird.

Verkürzt gesagt, das Set weist somit wenigstens zwei Elemente auf, nämlich die Nadelführungsvorrichtung und den Rechner mit einem Computerprogramm oder das Computerprogramm an sich. Das Computerprogramm kann auf einem Datenträger gespeichert sein. Das Set kann auch eines oder mehrere zusätzliche Elemente aufweisen, wie zum Beispiel die nachfolgend noch erläuterte Orientierungshilfe.

Ein solches Set unterstützt den Arzt bei der Durchführung der verschiedenen Schritte der Untersuchung und/oder Behandlung, zum Beispiel bei einer Biopsie. Insbesondere kann durch das Computerprogramm der Verfahrensablauf unterstützt werden, indem das Computerprogramm beziehungsweise der Rechner entsprechende Anleitungsschritte für die einzelnen durchzuführenden Verfahrensschritte ausgibt.

Der Rechner ist dazu eingerichtet, das Computerprogramm, z.B. im Sinne von Software, auszuführen. Der Rechner kann als handelsüblicher Computer ausgebildet sein, z.B. als PC, Laptop, Notebook, Tablet oder Smartphone, oder als Mikroprozessor, Mikrocontroller oder FPGA, oder als Kombination aus solchen Elementen.

Vorteilhafterweise unterstützt der Rechner beziehungsweise das Computerprogramm den Anwender insbesondere bei der Positionierung der Nadelführungsvorrichtung an dem Patienten und/oder bei der Einstellung der Winkelorientierung des Nadelführungselements gegenüber dem Basiselement in dem wenigstens einen Winkelfreiheitsgrad oder den mehreren voneinander unabhängigen Winkelfreiheitsgraden. Mit anderen Worten, durch das Computerprogramm kann dem Anwender das korrekte Positionieren und Einstellen der Nadelführungsvorrichtung deutlich erleichtert werden.

Das Computerprogramm benötigt als Input zumindest Kenndaten der Nadelführungsvorrichtung, zum Beispiel Angaben über die Geometrie eines Winkelverstellmechanismus und die Anordnung von Referenzpunkten des Basiselements. Die Kenndaten der Nadelführungsvorrichtung können beispielsweise im Rechner vorab gespeichert sein, wobei in dem Rechner bei Anwendung unterschiedlicher Nadelführungsvorrichtungen jeweils die Kenndaten dieser unterschiedlichen Nadelführungsvorrichtungen gespeichert sein können und dem Anwender eine Auswahl angezeigt werden kann, sodass der Anwender durch Eingabe an dem Rechner die jeweils eingesetzte Nadelführungsrichtung auswählen kann.

Das Computerprogramm benötigt als Input ferner Untersuchungsdaten des Patienten und wenigstens einen vorgegebenen mit der Nadelführungsvorrichtung durchzuführenden Untersuchungs- und/oder Behandlungsschritt. Die Untersuchungsdaten des Patienten können Daten aus einer bildgebenden Untersuchung des Patienten sein, beispielsweise aus einer Röntgen-basierten, MRT- und/oder Ultraschall-Untersuchung. Die Röntgen-basierte Untersuchung kann z.B. eine CT-Untersuchung sein. Beispielsweise können die hieraus gewonnenen Bilddaten beziehungsweise die DI-COM-Dateien direkt dem Rechner zur Verarbeitung mittels des Computerprogramms zugeführt werden. Die Angaben über den durchzuführenden Untersuchungs- oder Behandlungsschritt können zum Beispiel die von einem Anwender vorgegebenen Koordinaten eines Einstichpunkts der nadelförmigen Vorrichtung an dem Patienten sowie eines im Körper liegenden Zielpunkts sein.

Die erwähnten Input-Daten können dem Rechner über eine drahtlose oder drahtgebundene Schnittstelle und/oder über manuelle Eingaben zugeführt werden.

Das Computerprogramm erzeugt dann als Output die erwähnten Positionierungsangaben und/oder Winkelorientierungsangaben. Beispielsweise werden als Positionierungsangaben die zweidimensionalen Koordinaten in einem patientenbezogenen Koordinatensystem für wenigstens zwei Referenzpunkte, die sich beispielsweise am Basiselement der Nadelführungsvorrichtung befinden können, ausgegeben. Hinsichtlich der Winkelorientierungsangaben können zwei Winkelangaben ausgegeben werden, beispielsweise in der Einheit Grad, bezogen auf ein 360 Grad-System. Der Anwender kann dann mit diesen Positionierungsangaben und/oder Winkelorientierungsangaben die Nadelführungsvorrichtung an der gewünschten Stelle und in der gewünschten Lage an dem Körper platzieren und vor oder nach dieser Platzierung die Winkelorientierung des Nadelführungselements in den zwei Winkelfreiheitsgraden einstellen. Die in dem Set enthaltene Nadelführungsvorrichtung kann beispielsweise eine Nadelführungsvorrichtung der eingangs beschriebenen Art, das heißt eine erfindungsgemäße Nadelführungsvorrichtung, oder eine andersartige, beispielsweise eine bekannte Nadelführungsvorrichtung sein. So kann zum Beispiel auch die Nadelführungsvorrichtung gemäß der WO 2019/101 862 A1 verwendet werden.

Gemäß einer vorteilhaften Ausgestaltung der Erfindung ist vorgesehen, dass das Set als weiteres Element eine Orientierungshilfe aufweist, an dem Markierungen angebracht sind, die ein zweidimensionales Koordinatengitter definieren, wobei die Orientierungshilfe eine Befestigungsfläche aufweist, die dazu eingerichtet ist, die Orientierungshilfe an dem Patienten zu befestigen. Durch eine solche Orientierungshilfe wird die Zuordnung eines patientenbezogenen Koordinatensystems zu dem von dem Computerprogramm verwendeten Koordinatensystem sicherer und einfacher gestaltet. Insbesondere wird eine solche Zuordnung für den Anwender vereinfacht, da lediglich die Orientierungshilfe an dem Patienten angebracht werden muss und durch eine automatische Erfassung der Orientierungshilfe an dem Patienten, beispielsweise mittels einer Kamera und einer Bildauswertung, entsprechende Kenndaten des patientenbezogenen Koordinatensystems dem Rechner und dem Computerprogramm zur Verfügung gestellt werden können.

Die Orientierungshilfe kann zum Beispiel aus mehreren im rechten Winkel zueinander angeordneten Streifen bestehen, die zu einem Gitter zusammengefügt sind. An den einzelnen Streifen können Markierungen oder Skalen angebracht sein, die vom Anwender ablesbar sind und eine optische Erkennung einer gewünschten Koordinatenposition am Patienten erleichtern. Die Orientierungshilfe kann auch eine im Wesentlichen geschlossene Oberfläche aufweisen, ähnlich einem Blatt Papier oder einem großflächigen Pflaster, auf dem das zweidimensionale Koordinatengitter aufgebracht ist, beispielsweise durch Aufdrucken.

Auf der Orientierungshilfe kann das Koordinatengitter z.B. aufgedruckt oder in sonstiger Weise aufgebracht sein. Das Koordinatengitter kann im Sinne eines kartesischen Koordinatensystems gestaltet sein und kann z.B. eine horizontale Koordinate (X-Koordinate) und eine vertikale Koordinate (Y-Koordinate) haben. Die Beschriftung der Koordinatenachsen kann z.B. mit Zahlen und/oder Buchstaben erfolgen.

Gemäß einer vorteilhaften Ausgestaltung der Erfindung ist vorgesehen, dass die Orientierungshilfe als flaches flexibles Element ausgebildet ist. Dies hat den Vorteil, dass die Orientierungshilfe auch im Bereich von Körperrundungen und Ausbuchtungen problemlos am Patienten angebracht werden kann.

Gemäß einer vorteilhaften Ausgestaltung der Erfindung ist vorgesehen, dass die Untersuchungsdaten des Patienten Lageinformationen der am Patienten befestigten Orientierungshilfe beinhalten, wobei das Computerprogramm dazu eingerichtet ist, die Ausgabe der Positionierungsangaben und/oder der Winkelorientierungsangaben zusätzlich in Abhängigkeit von den Lageinformationen der Orientierungshilfe am Patienten zu berechnen. Dementsprechend beinhaltet der Input "Untersuchungsdaten" zusätzlich die Lageinformationen der am Körper der Patienten befestigten Orientierungshilfe. Diese Lageinformationen können vom Computerprogramm automatisch ausgewertet werden, sodass die Positionierungsangaben und/oder die Winkelorientierungsangaben unmittelbar in Bezug auf das durch die Orientierungshilfe definierte patientenbezogene Koordinatensystem ausgegeben werden können.

Gemäß einer vorteilhaften Ausgestaltung der Erfindung ist vorgesehen, dass die Orientierungshilfe mehrere Markerelemente aufweist, die anhand von Röntgen-basierten, MRT- und/oder Ultraschall-Bildern automatisch detektierbar sind. Diese Markerelemente können dabei räumlich verteilt an der Orientierungshilfe angeordnet sein. Beispielsweise können die Markerelemente matrixartig über die Orientierungshilfe verteilt angeordnet sein. Es können auch nur einzelne Punkte der Orientierungshilfe mit Markerelementen bestückt sein, beispielsweise zwei oder drei Punkte. Auch dies erlaubt bereits eine automatische Auswertung der Positionierung der Orientierungshilfe am Patienten anhand der Röntgen-basierten, MRT- und/oder Ultraschall-Bilder.

Ist eine größere Anzahl von Markerelementen vorhanden, die matrixartig verteilt sind, kann durch die Markerelemente zudem eine automatische Erkennung der Oberflächenkontur des Körpers des Patienten im Bereich der Orientierungshilfe anhand der Röntgen-basierten, MRT- und/oder Ultraschall-Bilder durchgeführt werden. Alternativ oder zusätzlich kann eine solche Erkennung der Oberflächenkontur auch unmittelbar durch Bildverarbeitung der Röntgen-basierten, MRT- und/oder Ultraschall-Bilder erfolgen, ohne dass eine solche Matrix von Markerelementen vorhanden ist. Eine automatische Ermittlung der Oberflächenkontur ist auch durch ein im Bereich des Patienten angeordnetes separates Erfassungsgerät möglich, beispielsweise mittels einer Kamera, einer stereoskopischen Kamera, eines Laserscanners oder vergleichbaren Erfassungsinstrumenten, wobei diese auch in Kombination miteinander genutzt werden können.

Die Eingangs genannte Aufgabe wird außerdem gelöst durch ein Computerprogramm für einen Rechner, wobei das Computerprogramm dazu eingerichtet ist, anhand von
- Kenndaten einer erfindungsgemäßen Nadelführungsvorrichtung zur Führung und Positionierung einer nadelförmigen Vorrichtung an dem Patienten, wobei die Nadelführungsvorrichtung ein Basiselement und ein Nadelführungselement aufweist, an dem die nadelförmige Vorrichtung entlang ihrer Längserstreckung führbar ist, wobei die Winkelorientierung des Nadelführungselements gegenüber dem Basiselement in zwei voneinander unabhängigen Winkelfreiheitsgraden einstellbar ist, wobei das Basiselement einen Grundkörper aufweist, der eine Nadeldurchführöffnung aufweist und wobei der Grundkörper mehrere Positionsmarkierungen aufweist, wobei mittels dieser Positionsmarkierungen eine präzise Positionierung der Nadelführungsvorrichtung entsprechend einem patientenbezogenen Koordinatensystem am Patienten erfolgen kann,
- Untersuchungsdaten des Patienten aus einer bildgebenden Untersuchung des Patienten und
- wenigstens eines vorgegebenen, mit der Nadelführungsvorrichtung durchzuführenden Untersuchungs- und/oder Behandlungsschritts
   b1) Positionierungsangaben zur Positionierung der Nadelführungsvorrichtung an dem Patienten und
   b2) eine oder mehrere Winkelorientierungsangaben zur Einstellung der Winkelorientierung des Nadelführungselements gegenüber dem Basiselement in dem wenigstens einen Winkelfreiheitsgrad oder den mehreren voneinander unabhängigen Winkelfreiheitsgraden
      zu berechnen und dem Anwender auszugeben, wenn das Computerprogramm auf dem Rechner ausgeführt wird.

Auch hierdurch können die zuvor erläuterten Vorteile realisiert werden.

Die Erfindung wird nachfolgend anhand von Ausführungsbeispielen unter Verwendung von Zeichnungen näher erläutert.

Es zeigen:
- Figur 1: eine Nadelführungsvorrichtung in Explosionsdarstellung,
- Figur 2: ein Basiselement,
- Figur 3, 4: einen Haltebügel,
- Figur 5: einen Klemmbügel,
- Figur 6: ein Nadelführungselement,
- Figur 7: eine Nadelführungselementaufnahme,
- Figur 9: einen Halteschlitten,
- Figur 10: ein Bedienelement,
- Fig. 11,12: die Nadelführungsvorrichtung in unterschiedlichen Winkeleinstellungen,
- Figur 13: einen Rechner mit einem Computerprogramm und
- Figur 14: eine Orientierungshilfe und
- Figur 15: eine weitere Ausführungsform einer Orientierungshilfe und
- Figur 16: eine weitere Ausführungsform eines Haltebügels und
- Figur 17: eine stark schematisierte Darstellung des Haltebügels und des Klemmbügels und
- Figur 18: die Nadelführungsvorrichtung in verschiedenen Winkeleinstellungen und
- Figur 19: eine Erläuterung des Klemmmechanismus.

Die in den Figuren verwendeten Bezugszeichen haben folgende Zuordnung:
- 1: Nadelführungsvorrichtung
- 2: Nadelführungselement
- 3: Bedienelement
- 4: Halteschlitten
- 5: Haltebügel
- 6: Klemmbügel
- 7: Basiselement
- 8: Kontaktelement
- 9: Nadelführungselementaufnahme
- 10: nadelförmige Vorrichtung
- 11: Spitze
- 20: Nadelführungskörper
- 21: Durchgangskanal
- 22: Verbindungsbereich
- 23: Handhabungsbereich
- 24: Griffmulden
- 25: Einführungsöffnung
- 30: Griffelemente
- 31: Hohlraum
- 32: Innengewinde
- 40: Grundkörper
- 41: Öffnung
- 42: zweiter Winkelzeiger
- 50: Grundkörper
- 51: Öffnung
- 52: Lagerelement
- 53: Außenoberfläche
- 54: Randbereich
- 55: erster Winkelzeiger
- 56: zweite Winkelskala
- 60: Grundkörper
- 61: Öffnung
- 62: Lagerelement
- 70: Grundkörper
- 71: Nadeldurchführöffnung
- 72: Positionsmarkierung
- 73: Markierung
- 74: Klemmbogen
- 75: Lagerbogen
- 76: Aussparung
- 77: Haltebogen
- 78: Aussparung
- 79: erste Winkelskala
- 90: Längsabschnitt
- 91: Durchgangsöffnung
- 92: Bereich
- 93: Lagerungsfläche
- 94: Gewindebereich
- 95: Halteabschnitt
- 96: Fixierelement
- 100: Rechner
- 101: Computerprogramm
- 102: Kenndaten
- 103: Untersuchungsdaten
- 104: Daten eines Untersuchungs- und/oder Behandlungsschritts
- 105: Positionierungsangaben
- 106: Winkelorientierungsangaben
- 200: Orientierungshilfe
- 201: gitterförmige Struktur
- 202: Markierung
- 203: Markerelement

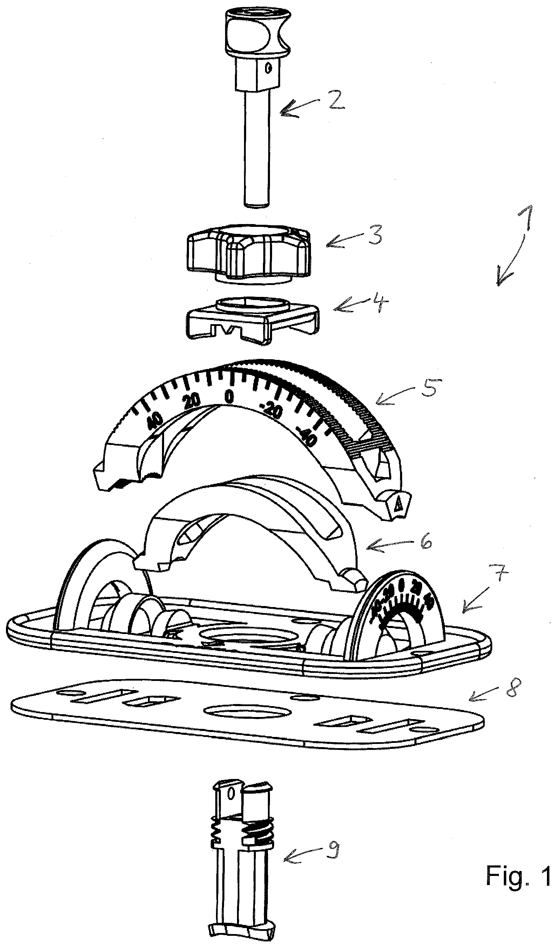

Die in Figur 1 dargestellte Nadelführungsvorrichtung 1 weist ein Nadelführungselement 2, ein Bedienelement 3, einen Halteschlitten 4, einen Haltebügel 5, einen Klemmbügel 6, ein Basiselement 7, ein Kontaktelement 8, sowie eine Nadelführungselementaufnahme 9 auf. Das Kontaktelement 8 ist optional. Es dient beispielsweise zur Befestigung des Basiselements 7 am Körper eines Patienten. Zu diesem Zweck kann das Kontaktelement 8 beispielsweise mit einem Klebstoff versehen sein. Alternativ kann die Unterseite des Basiselements 7 direkt mit dem Körper des Patienten in Kontakt gebracht werden. In diesem Fall kann dort ein Klebstoff angeordnet sein. Insgesamt kann die Nadelführungsvorrichtung 1 damit aus nur sieben Bauteilen 2, 3, 4, 5, 6, 7 und 9 realisiert sein, die ganz oder überwiegend als Kunststoffbauteile ausgebildet sein können.

Diese Bauteile werden nachfolgend der Reihe nach erläutert.

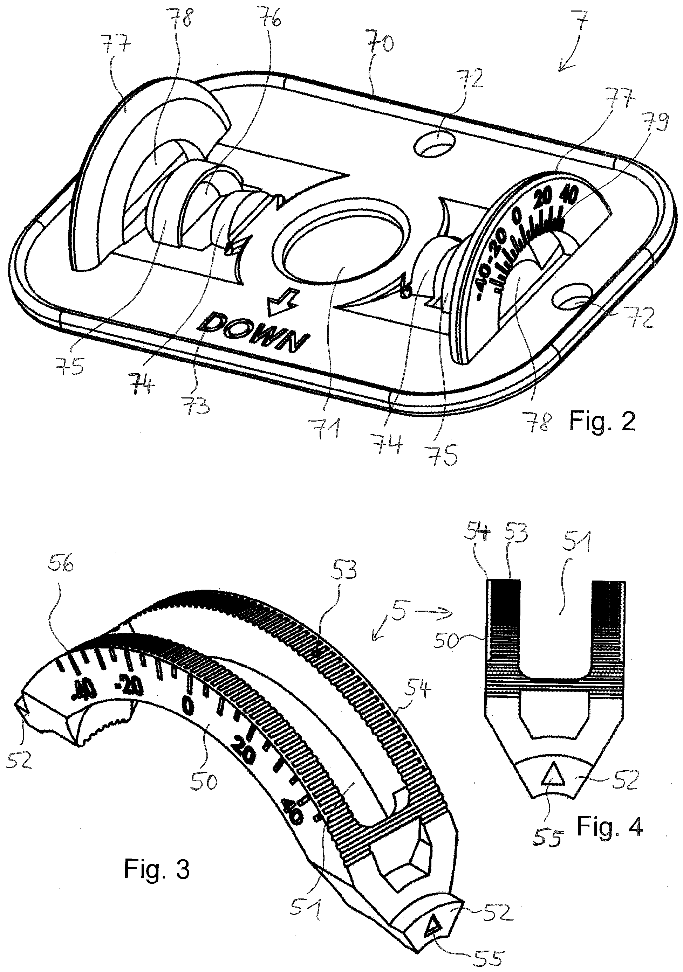

Die Figur 2 zeigt zunächst das Basiselement 7 in einer perspektivischen Ansicht. Das Basiselement 7 weist einen Grundkörper 70 auf, der nach Art einer flachen, ebenen Platte ausgebildet sein kann. An der Unterseite des Grundkörpers 70 kann das Basiselement 7 eine Auflagefläche zur Auflage an dem Körper des Patienten aufweisen. Dort kann auch das erwähnte Kontaktelement 8 angebracht sein.

Der Grundkörper 70 weist eine Nadeldurchführöffnung 71 auf, die zur Durchführung der nadelförmigen Vorrichtung zum Körper des Patienten dient. Im Bereich der Nadeldurchführöffnung 71 kann zum Beispiel bei einer Biopsie die Einstichstelle der Biopsienadel angeordnet sein. Ferner weist der Grundkörper 70 mehrere Positionsmarkierungen 72 auf, beispielsweise in Form von Durchgangslöchern. Mittels dieser Positionsmarkierungen 72 kann eine präzise Positionierung der Nadelführungsvorrichtung 1 entsprechend einem patientenbezogenen Koordinatensystem am Patienten erfolgen.

Zusätzlich kann der Grundkörper 70 weitere Markierungen 73 aufweisen, zum Beispiel Angaben zu einer Bezugslage, beispielsweise der Unterseite der Nadelführungsvorrichtung 1.

Auf beiden Seiten der Nadeldurchführöffnung 71 ragen von dem Grundkörper 70 Haltebögen 77 ab, die zum Halten und für die Schwenklagerung des Haltebügels 5 an dem Basiselement 7 dienen. Die Haltebögen 77 weisen jeweilige kreisbogenförmige beziehungsweise halbkreisförmige Aussparungen 78 auf, an denen der Haltebügel 5 verschwenkbar gelagert werden kann. Benachbart zu einem jeweiligen Haltebogen 77 ragt von dem Grundkörper 70 jeweils ein Lagerbogen 75 ab. Die nach außen gewandte gekrümmte Oberfläche des Lagerbogens 75 dient zusätzlich zur Bildung eines Schwenklagers für den Haltebügel 5. Die Lagerbögen 75 weisen ebenfalls innere Aussparungen 76 auf, die als Teil eines Schwenklagers des Klemmbügels 6 dienen. Benachbart zu den Lagerungsbögen 75, z.B. auf jeder Seite der Nadeldurchführöffnung 71, ist an dem Grundkörper 70 jeweils ein Klemmbogen 74 angeordnet. Die gekrümmte Außenoberfläche des Klemmbogens 74 dient als weiterer Teil des Schwenklagers für den Klemmbügel 6. Wie man erkennt, befindet sich ausgehend von der Mitte des Grundkörpers 70 beziehungsweise von der Nadeldurchführöffnung 71 aus zunächst auf jeder Seite ein Klemmbogen 74, daraufhin ein Lagerbogen 75 und schließlich ein Haltebogen 77.

Erkennbar ist ferner, dass an der von der Nadeldurchführöffnung 71 abgewandten Oberfläche des Haltebogens 77 eine erste Winkelskala 79 angeordnet sein kann.

Die Figur 3 zeigt den Haltebügel 5 in einer perspektivischen Ansicht, die Figur 4 in einer Seitenansicht. Der Haltebügel 5 weist einen Grundkörper 50 auf, der bogenförmig ausgebildet ist und eine schlitzartige, in Längsrichtung verlaufende Öffnung 51 aufweist, durch die das Nadelführungselement 2 beziehungsweise die Nadelführungselementaufnahme 9 geführt werden kann. Der Haltebügel 5 weist an den beiden voneinander entfernten freien Enden jeweils ein Lagerelement 52 auf, das hinsichtlich seiner gekrümmten Außenkonturen an die Innenkontur der Öffnung 78 und an die zum Haltebogen 77 gewandte Außenkontur des Lagerbogens 75 angepasst ist. Auf diese Weise kann der an dem Basiselement 7 mit seinem Lagerelement 52 zwischen dem Haltebogen 77 und dem Lagerbogen 75 eingesetzte Haltebügel 5 stufenlos um eine auf diese Weise gebildete Schwenkachse verschwenkt werden. An dem Lagerelement 52 ist ein erster Winkelzeiger 55 angeordnet, der der ersten Winkelskala 79 zugeordnet ist. Durch die Anzeige des ersten Winkelzeigers 55 auf der ersten Winkelskala 79 kann die Winkelorientierung des Nadelführungselements 2 gegenüber des Basiselement 7 in einem ersten Winkelfreiheitsgrad abgelesen werden.

Erkennbar ist in den Figuren 3 und 4 zusätzlich, dass der Haltebügel 5 an den konvex gewölbten Außenoberflächen 53 eine Strukturierung, zum Beispiel eine Riffelung, aufweisen kann. Die Außenoberflächen 53 dienen als Auflage- und Gleitfläche für den Halteschlitten 4. Um dennoch eine möglichst stufenlose Einstellung des Halteschlittens 4 auf den Außenoberflächen 53 zu gewährleisten, kann die Oberflächenstrukturierung beziehungsweise die Riefelung etwas versenkt gegenüber einem Randbereich 54 der Außenoberflächen 53 angeordnet sein, sodass die Oberflächenstrukturierung beziehungsweise Riffelung erst bei einer Fixierung des Klemmmechanismus wirksam wird.

Erkennbar ist ferner, dass an dem Haltebügel 5 eine zweite Winkelskala 56 angeordnet sein kann.

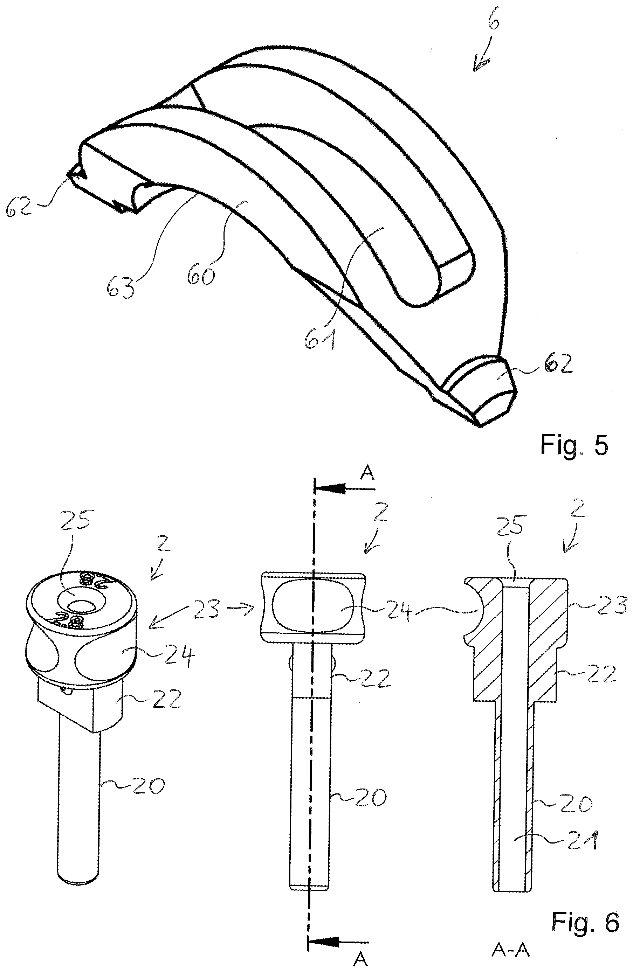

Die Figur 5 zeigt den Klemmbügel 6 in einer perspektivischen Darstellung. Der Klemmbügel 6 ist ähnlich geformt wie der Haltebügel 5. Der Klemmbügel 6 weist einen Grundkörper 60 auf, der bogenförmig verläuft und eine schlitzartige, in Längsrichtung verlaufende Öffnung 61 aufweist. Durch die schlitzartige Öffnung 61 kann das Nadelführungselement 2 beziehungsweise die Nadelführungselementaufnahme 9 geführt werden. An der konkaven Innenoberfläche 63 des bogenförmigen Grundkörpers 60 ist eine Haltefläche ausgebildet, die als Gegenlager für die Nadelführungselementaufnahme 9 dient, wie nachfolgend noch erläutert wird.

Ähnlich wie der Haltebügel 5 weist der Klemmbügel 6 an den voneinander entfernten freien Enden jeweils ein Lagerelement 62 auf, mit dem der Klemmbügel 6 in der Öffnung 76 des Basiselements 7 platziert werden kann. Das Lagerelement 62 befindet sich dann zwischen einer Innenseite des Lagerbogens 75 und einer Außenoberfläche des Klemmbogens 74. Auf diese Weise wird ein Schwenklager zur Verschwenkung des Klemmbügels 6 gebildet. Dabei sind die Schwenkachsen des Klemmbügels 6 und des Haltebügels 5 identisch.

Die Figur 6 zeigt das Nadelführungselement 2 links in einer perspektivischen Darstellung, in der Mitte in einer Seitenansicht und rechts in einer Schnittansicht in der Schnittebene A-A. Das Nadelführungselement 2 weist einen Nadelführungskörper 20 auf, der beispielsweise als hohlzylindrischer Körper mit einem inneren Durchgangskanal 21 ausgebildet sein kann. Der innere Durchgangskanal 21 erstreckt sich in Längsrichtung durch den Nadelführungskörper 20 vollständig hindurch. Durch den Durchgangskanal 21 kann die nadelförmige Vorrichtung, zum Beispiel eine Biopsienadel, hindurchgeführt werden. Das Nadelführungselement 2 weist oberhalb des Nadelführungskörpers 20 einen Verbindungsbereich 22 auf, in dem das Nadelführungselement 2 mit der Nadelführungselementaufnahme 9 gekoppelt werden kann.

Oberhalb des Verbindungsbereichs 22 weist das Nadelführungselement 2 einen manuellen Handhabungsbereich 23 auf, der beispielsweise eine oder mehrere Griffmulden 24 aufweisen kann, mit denen das Greifen und manuelle Einsetzen oder Lösen des Nadelführungselements 2 von der Nadelführungsvorrichtung 1 vereinfacht wird. Der innere Durchgangskanal 21 erstreckt sich in Längsrichtung auch durch den Verbindungsbereich 22 und den manuellen Handhabungsbereich 23 hindurch. Am oberen Ende des Nadelführungselements 2 mündet der innere Durchgangskanal 21 in eine Einführöffnung 25, an der die nadelförmige Vorrichtung in das Nadelführungselement 2 eingeführt werden kann.

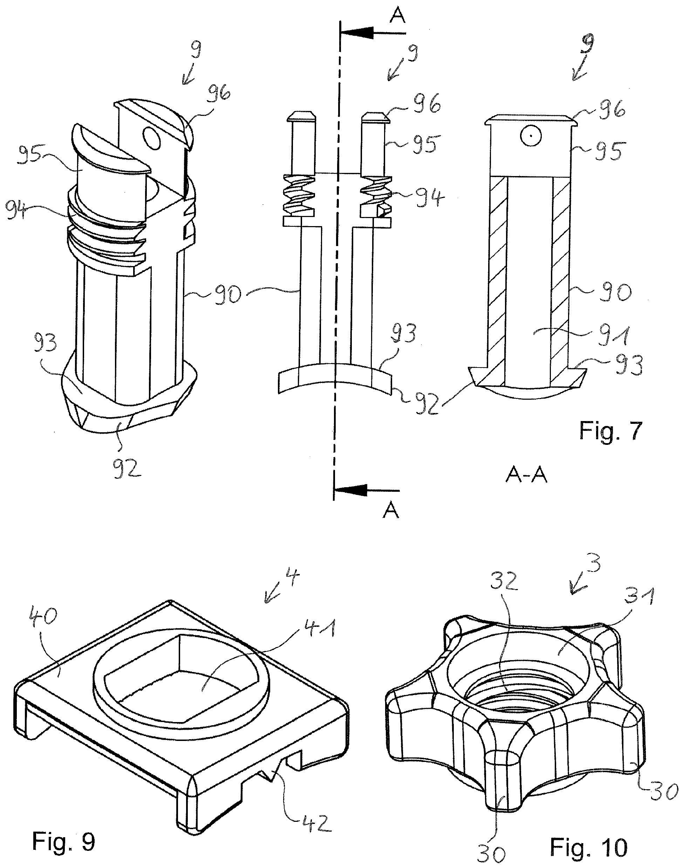

Die Figur 7 zeigt die Nadelführungselementaufnahme 9 links in einer perspektivischen Darstellung, in der Mitte in einer Seitenansicht und rechts in einer Schnittansicht in der Schnittebene A-A. Die Nadelführungselementaufnahme 9 dient zur Aufnahme unterschiedlich ausgebildeter Nadelführungselemente 2. Als gemeinsame Schnittstelle zu den unterschiedlich ausgebildeten Nadelführungselementen 2 weist die Nadelführungselementaufnahme 9 eine in Längsrichtung verlaufende Durchgangsöffnung 91 auf, die hinsichtlich ihrer Abmessungen an die Außenabmessungen des Nadelführungskörpers 20 des Nadelführungselements 2 angepasst ist. Das Nadelführungselement 2 wird somit mit dem Nadelführungskörper 20 in die Durchgangsöffnung 91 eingesetzt. Ist das Nadelführungselement 2 vollständig in die Nadelführungselementaufnahme 9 eingesetzt, so befindet sich der Verbindungsbereich 22 innerhalb eines Halteabschnitts 95 der Nadelführungselementaufnahme 9, an dem das Nadelführungselement 2 an der Nadelführungselementaufnahme 9 fixiert wird. Der Halteabschnitt 95 kann in Form zweier in Längsrichtung abragender Haltelaschen ausgebildet sein, die umfangsseitig zumindest teilweise kreisbogenförmig sind. Durch diese kreisbogenförmige Umfangskontur wird zugleich eine Drehlagerung für das Bedienelement 3 bereitgestellt.

Der Halteabschnitt 95 kann am freien Ende mit einem umfangsseitig abstehenden Fixierelement 96 enden, beispielsweise ähnlich einer Pilzkopfform. Hierdurch kann das Bedienelement 3 unverlierbar mit der Nadelführungselementaufnahme 9 gekoppelt werden. Das Bedienelement 3 wird einfach durch Aufstecken auf den Halteabschnitt 95 daran verrastet und durch die abragenden Fixierelemente 96 daran fixiert.

Unterhalb des Halteabschnitts 95 weist die Nadelführungselementaufnahme 9 einen Gewindebereich 94 auf, der als ein zu einem Innengewinde des Bedienelements 3 passendes Gewinde ausgebildet ist. Unterhalb des Gewindebereichs 94 befindet sich ein Längsabschnitt 90 der Nadelführungselementaufnahme 9, der im fertig montierten Zustand der Nadelführungsvorrichtung 1 eine Verbindung zwischen dem Haltebügel 5 und dem Klemmbügel 6 herstellt. Unterhalb des Längsabschnitts 90 geht die Nadelführungselementaufnahme 9 in einen Bereich 92 mit vergrößerter Querschnittsfläche über, der zumindest in einer Betrachtungsrichtung (Figur 7 Mitte) eine bogenförmige Lagerungsfläche 93 ausbildet. Im fertig montierten Zustand der Nadelführungsvorrichtung 1 kommt die bogenförmige Lagerungsfläche 93 in Kontakt mit der konkaven Innenoberfläche 63 des Klemmbügels 6. Auf diese Weise wird ein Teil der beweglichen Schwenklagerung des Nadelführungselements 2 an dem Haltebügel 5 und dem Klemmbügel 6 gebildet.

Die Figur 9 zeigt den Halteschlitten 4 in einer perspektivischen Darstellung. Der Halteschlitten 4 weist einen Grundkörper 40 auf, der eine Öffnung 41 aufweist. Durch die Öffnung 41 kann ein Teil der Nadelführungselementaufnahme 9 hindurch gesteckt werden, insbesondere der obere Teil des Längsabschnitts 90, der in den Gewindebereich 94 übergeht. Der Halteschlitten 4 weist an der Unterseite Auflageflächen auf, über die der Halteschlitten 4 auf den konvex gewölbten Außenoberflächen 53 des Haltebügels 5 geführt ist und darauf abgleitet oder, bei Aktivierung des Klemmmechanismus, darauf festgeklemmt ist. An dem Halteschlitten 4 ist ferner der zweite Winkelzeiger 42 angeordnet, der zur Winkelanzeige auf der zweiten Winkelskala 56 des Haltebügels 5 dient.

Die Figur 10 zeigt das Bedienelement 3, das in Form einer Mutter mit einem Innengewinde 32 ausgebildet sein kann. Das Innengewinde 32 kann auf den Gewindebereich 94 der Nadelführungselementaufnahme 9 geschraubt werden. Das Bedienelement 3 weist einen inneren Hohlraum 31 auf, durch den zumindest ein Teil des Nadelführungselements 2 hindurch gesteckt werden kann. Am Außenumfang kann das Bedienelement 3 Griffelemente 30 aufweisen, die zur Verbesserung der Übertragung einer Bedienkraft geeignet sind, wie zum Beispiel die dargestellten Vorsprünge oder eine sonstige Oberflächenstrukturierung und/oder Riffelung am Außenumfang.

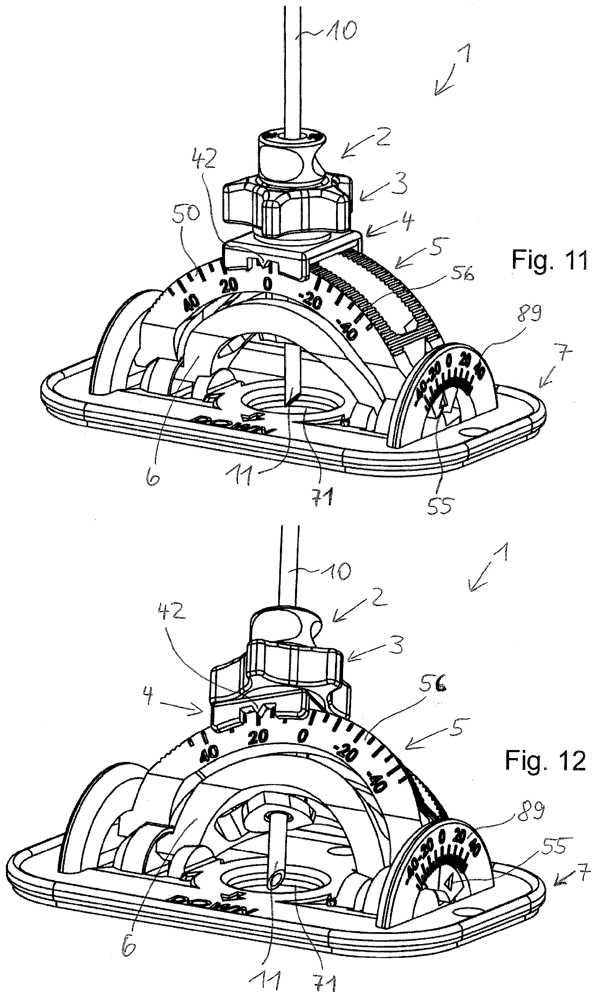

Die Figur 11 zeigt die zusammengebaute Nadelführungsvorrichtung 1 mit den bereits erwähnten Elementen, zudem ist eine nadelförmige Vorrichtung 10 zumindest teilweise dargestellt. Die nadelförmige Vorrichtung 10 endet im Bereich der Durchgangsöffnung 71 des Basiselements 7 mit einer Spitze 11. Die Figur 11 zeigt die Nadelführungsvorrichtung 1 in einer Einstellung, in der die Winkelorientierung des Nadelführungselements 2 bezüglich beider einstellbaren Winkelfreiheitsgrade neutral ist, das heißt die jeweiligen Winkelzeiger 42, 55 zeigen auf den Null-Wert der jeweiligen Winkelskala 56, 79. Bei der Figur 12 wurde eine Verstellung der Winkelorientierung durchgeführt, derart, dass zum Beispiel der zweite Winkelzeiger 42 auf einen Winkel von ca. -20 Grad an der zweiten Winkelskala 56 zeigt. Der erste Winkelzeiger 55 zeigt auf einen Winkel von ca. +30 Grad auf der ersten Winkelskala 79.

Für die Einstellung der Nadelführungsvorrichtung 1 von einer Winkelorientierung auf eine andere Winkelorientierung muss lediglich das Bedienelement 3 geringfügig um seine Längsachse gedreht werden, beispielsweise in Linksdrehrichtung, wodurch die Gewindeverbindung zwischen dem Innengewinde 32 und dem Gewindebereich 94 etwas verstellt wird und hierdurch durch die durch den Klemmbügel 6, der über die Gewindeverbindung und die Nadelführungselementaufnahme 9 verspannt werden kann, bewirkte Klemmung aufgehoben wird. Ist die gewünschte Winkelorientierung eingestellt, muss lediglich das Bedienelement 3 in entgegengesetzter Drehrichtung verdreht werden, wodurch über die Gewindeverbindung 32, 94 eine Spannkraft über die Nadelführungselementaufnahme 9 auf den Klemmbügel 6 ausgeübt wird und dieser gegenüber dem Haltebügel 5 verspannt wird. Hierdurch werden sämtliche Verschwenk- oder Bewegungsfreiheitsgrade der Winkelverstellung der Nadelführungsvorrichtung 1 fixiert.

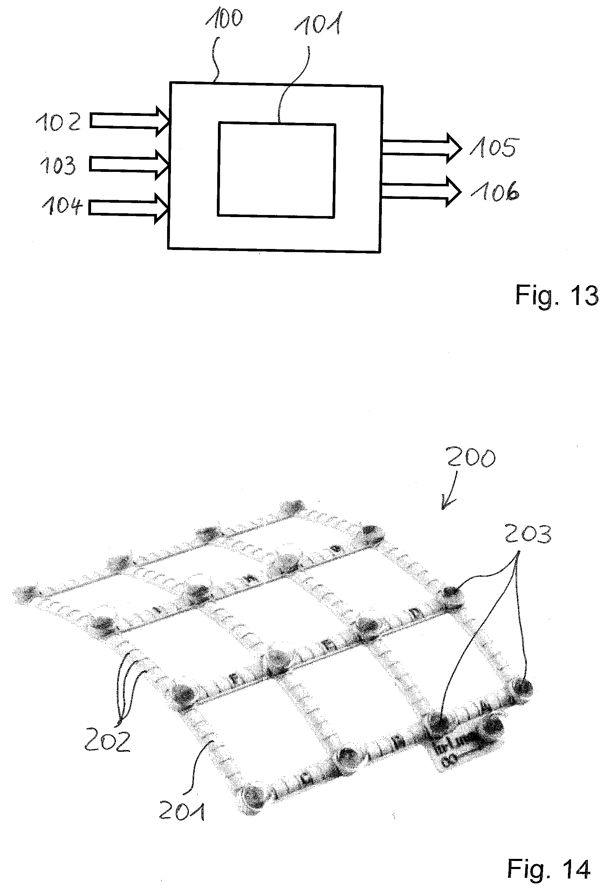

Die Figur 13 zeigt als Teil eines Sets mit mehreren Elementen für die Durchführung einer Untersuchung und/oder Behandlung an einem Körper eines Patienten einen Rechner 100, der ein Computerprogramm 101 aufweist. Zu dem Set kann eine beliebige Nadelführungsvorrichtung gehören, beispielsweise eine Nadelführungsvorrichtung der zuvor beschriebenen Art, und/oder eine Orientierungshilfe der zuvor beschriebenen Art.

Im Rechner 100 beziehungsweise im Computerprogramm 101 werden als Eingangsdaten Kenndaten 102 der im Set enthaltenen Nadelführungsvorrichtung, Untersuchungsdaten 103 eines Patienten und Daten 104 eines vorgegebenen, mittels der Nadelführungsvorrichtung durchzuführenden Untersuchungs- und/oder Behandlungsschritts zugeführt. Hieraus berechnet das Computerprogramm Positionierungsangaben 105 zur Positionierung der Nadelführungsvorrichtung an dem Patienten sowie Winkelorientierungsangaben 106 zur Einstellung der Winkelorientierung des Nadelführungselements gegenüber dem Basiselement in den zwei voneinander unabhängigen Winkelfreiheitsgraden. Die ausgegebenen Positionierungsangaben und Winkelorientierungsangaben können beispielsweise auf einem Display des Rechners 100 dargestellt werden.

Das erwähnte Set kann eine Orientierungshilfe 200 aufweisen, beispielsweise die in Figur 14 dargestellte Orientierungshilfe 200. Die Orientierungshilfe 200 weist eine gitterförmige Struktur 201 auf, die aus flachen, flexiblen Teilelementen gebildet sein kann. An diesen Teilelementen können Markierungen 202 zur Definition eines zweidimensionalen Koordinatengitters angeordnet sein. Zudem können an mehreren Stellen, beispielsweise matrixförmig, Markerelemente 203 an der Orientierungshilfe 200 angeordnet sein. Die Markerelemente 203 sind aus einem Material ausgebildet, das bei Röntgen-basierten, MRT- und/oder Ultraschall-Untersuchungen erkannt werden kann und dementsprechend auch durch Bildverarbeitung automatisch ausgewertet werden kann.

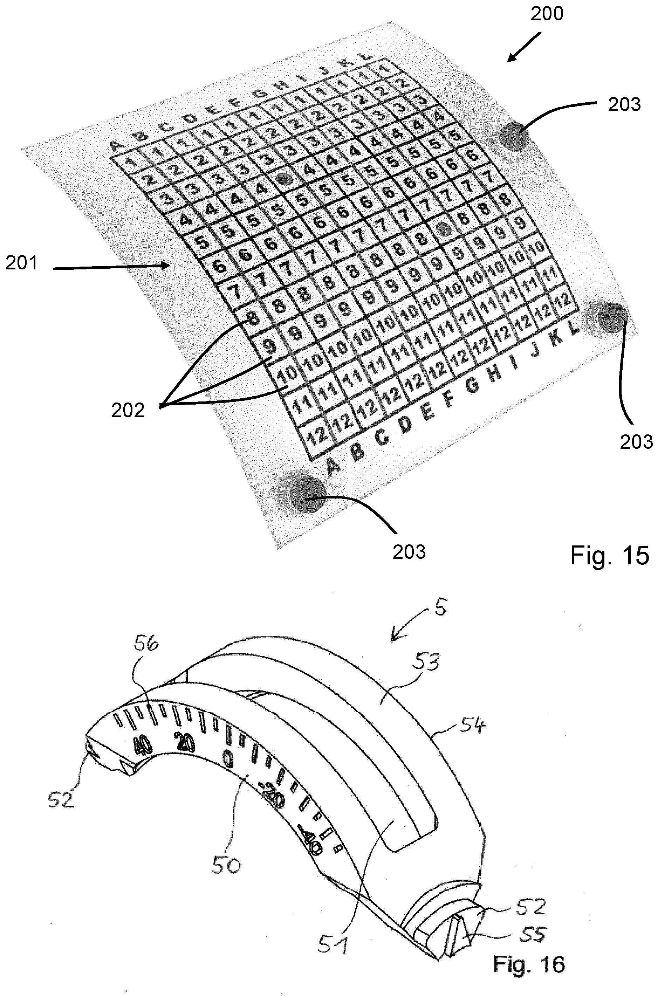

Die Figur 15 zeigt eine weitere Ausführungsform einer Orientierungshilfe 200. Während die Ausführungsform gemäß Figur 14 eine gitterförmige Struktur aus überkreuz miteinander verbundenen länglichen Elementen aufweist, zeigt die Figur 15 eine Ausführungsform, bei der die gitterförmige Struktur 201 auf einem zumindest im Wesentlichen geschlossen ausgebildeten Grundkörper, z.B. auf einem Blatt Papier oder einer Folie, aufgebracht ist. Die gitterförmige Struktur 201 kann zusammen mit den daran angeordneten Markierungen 202 beispielsweise aufgedruckt sein. Auch bei dieser Orientierungshilfe 200 können die erwähnten Markerelemente 203 vorhanden sein.

Sofern das erwähnte selbst nur die Orientierungshilfe 200 und dem Rechner 100 bzw. das Computerprogramm 101 aufweist, kann das Computerprogramm 101 derart gestaltet sein, dass als Eingangsdaten lediglich Kenndaten der Orientierungshilfe 200, Untersuchungsdaten des Patienten und wenigstens eine vom Anwender in den Untersuchungsdaten des Patienten ausgewählte Position am Patienten notwendig sind. Das Computerprogramm 101 berechnet hieraus Koordinatenangaben im Koordinatengitter der Orientierungshilfe und gibt diese dem Anwender aus, z.B. die Angabe E 4 für eine Position in der vierten Zeile und der fünften Spalte.

Die Figur 16 zeigt eine alternative Ausführungsform des Haltebügels 5, bei dem im Unterschied zu der bisher beschriebenen Ausführungsform an den konvex gewölbten Außenoberflächen 53 keine besondere Strukturierung vorhanden ist, sondern diese Außenoberflächen 53 mit der für das verwendete Material normalen, relativ glatten Oberfläche ausgebildet sind.

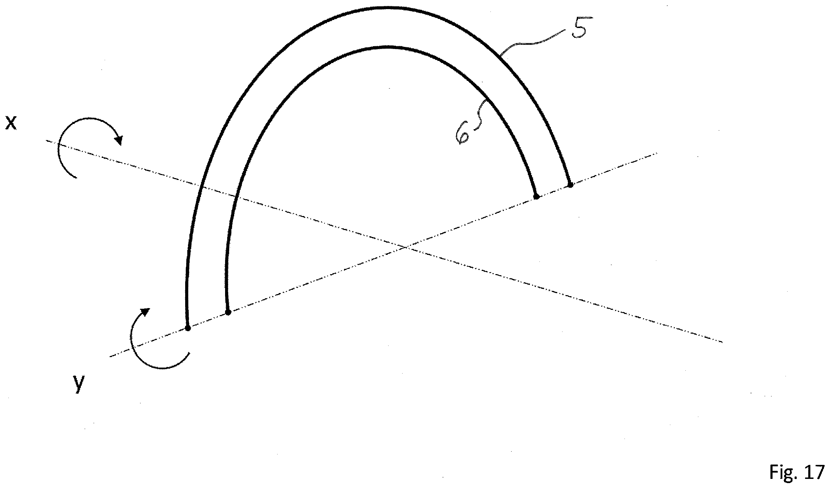

Die Figur 17 zeigt stark schematisiert den Haltebügel 5 und den Klemmbügel 6 sowie die zueinander orthogonalen Verschwenkachsen X, Y, in denen die Nadelführungsvorrichtung 1 in verschiedenen Winkelorientierungen einstellbar ist. Man erkennt, dass der Haltebügel 5 und der Klemmbügel 6 jeweils im Wesentlichen bogenförmig ausgebildet sind, wobei diese Bögen im Wesentlichen parallel zueinander verlaufen. Der Haltebügel 5 befindet sich dabei über dem Klemmbügel 6. Sowohl der Haltebügel 5 als auch der Klemmbügel 6 ist um die Y-Achse verschwenkbar. Da in diesem Bereich kein weiterer Bogen für die Verschwenkbarkeit um die X-Achse vorhanden ist, ist unterhalb des Klemmbügels 6 ein Freiraum vorhanden.

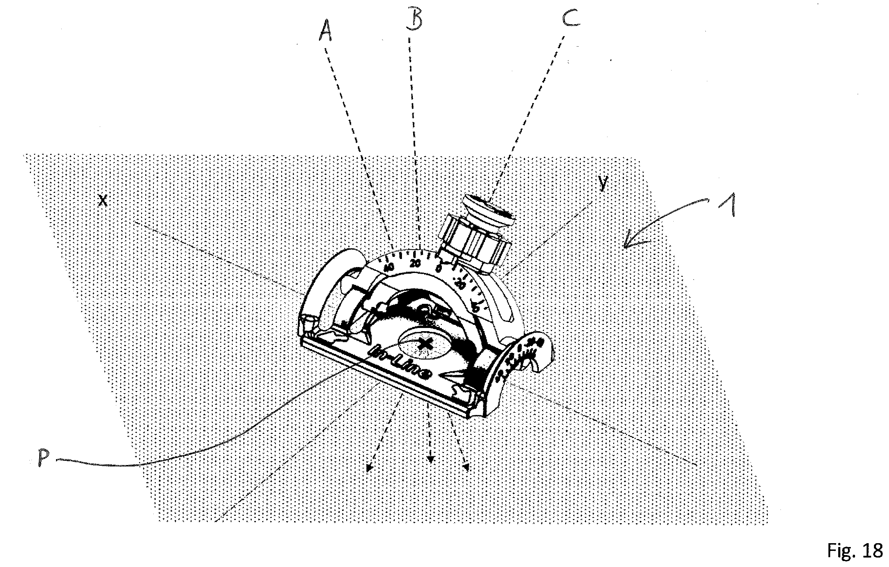

Die Figur 18 verdeutlicht anhand von drei verschiedenen Winkelorientierungen A, B, C, in die die Nadelführungsvorrichtung 1 eingestellt werden kann, dass bei jeder Einstellung die nadelförmige Vorrichtung 10 durch das Nadelführungselement 2 immer durch denselben Punkt P geführt wird, d. h. es ergibt sich für die nadelförmige Vorrichtung 10 immer dieselbe Einstichstelle am Patienten, unabhängig von der Winkeleinstellung.

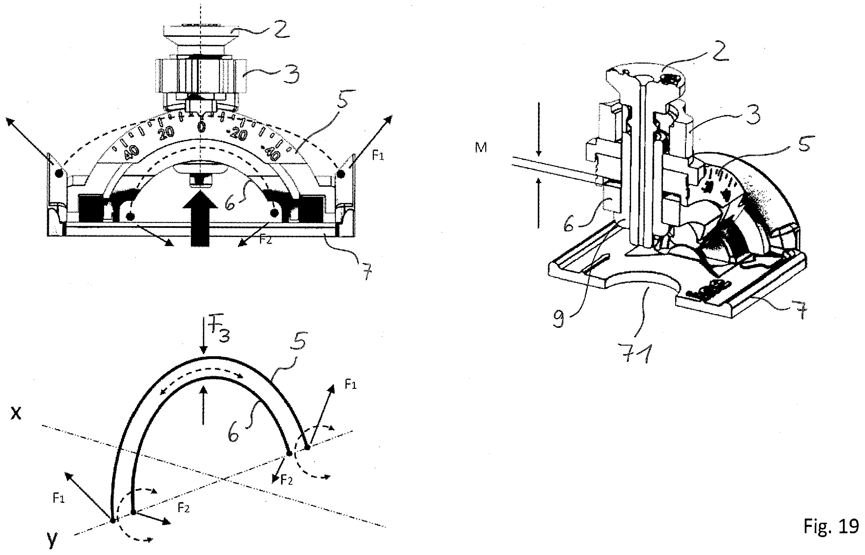

Die Figur 19 verdeutlicht anhand von Teildarstellungen der Nadelführungsvorrichtung 1 (oben) sowie der schematischen Darstellung ähnlich Figur 17 (unten) die Funktionsweise des Klemmmechanismus. Die Klemmung wird über das Bedienelement 3 hervorgerufen, d. h. durch Festziehen der Mutter mit dem Innengewinde 32 gegenüber dem Gewindebereich 94 der Nadelführungselementaufnahme 9. Hierdurch wird der Haltebügel 5 gegenüber dem Klemmbügel 6 verspannt, d. h. durch das Festziehen wird der Klemmbügel 6 etwas zum Haltebügel 5 herangezogen. Der dargestellte Abstand M wird hierbei verringert. Die Bogenform des Haltebügels 5 wird hierdurch etwas abgeflacht, während der Klemmbügel 6 etwas stärker durchgebogen wird. Hierdurch ist zunächst das Nadelführungselement 2 im Hinblick auf die Verschwenkbarkeit um die X-Achse fixiert. Zudem ergibt sich eine Fixierung des Haltebügels 5 und des Klemmbügels 6 an dem Basiselement 7 durch die in diesem Übergangsbereich auftretenden Kräfte F₁, F₂.

## Patentansprüche

1. Nadelführungsvorrichtung (1) zur Führung und Positionierung einer nadelförmigen Vorrichtung (10) an einem Patienten, wobei die Nadelführungsvorrichtung (1) ein Basiselement (7) und ein Nadelführungselement (2) aufweist, an dem die nadelförmige Vorrichtung (10) entlang ihrer Langserstreckung führbar ist, wobei die Winkelorientierung des Nadelführungselements (2) gegenüber dem Basiselement (7) in zwei voneinander unabhängigen Winkelfreiheitsgraden einstellbar ist, mit folgenden Merkmalen:
a) die Nadelführungsvorrichtung (1) weist ein Bedienelement (3) auf, durch das die Winkelorientierung des Nadelführungselements (2) in den zwei Winkelfreiheitsgraden fixierbar oder eine solche Fixierung wieder lösbar ist,
b) die Nadelführungsvorrichtung (1) weist für jeden der zwei einstellbaren Winkelfreiheitsgrade eine Winkelskala und einen der jeweiligen Winkelskala (56, 89) zugeordneten Winkelzeiger (42, 55) auf, sodass durch den Anwender eine aktuell eingestellte Winkelorientierung des Nadelführungselements (2) in den zwei Winkelfreiheitsgraden anhand der Position des jeweiligen Winkelzeiger (42, 55) gegenüber der zugeordneten Winkelskala (56, 89) ablesbar ist,
wobei die Nadelführungsvorrichtung (1) einen Haltebügel (5) aufweist, an dem das Nadelführungselement (2) befestigt ist, wobei der Haltebügel (5) in einem ersten der zwei Winkelfreiheitsgrade gegenüber dem Basiselement (7) bewegbar ist und das Nadelführungselement (2) in einem zweiten der zwei Winkelfreiheitsgrade gegenüber dem Haltebügel (5) bewegbar ist, **dadurch gekennzeichnet, dass** die Nadelführungsvorrichtung (1) einen Klemmbügel (6) aufweist, der an dem Basiselement (7) befestigt ist und um dieselbe Schwenkachse gegenüber dem Basiselement (7) verschwenkbar ist wie der Haltebügel (5).

2. Nadelführngsvorrichtung (1) nach Anspruch 1, **dadurch gekennzeichnet, dass** die Nadelführungsvorrichtung (1) einen durch das Bedienelement (3) betätigbaren Klemmmechanismus aufweist, wobei die Winkelorientierung des Nadelführungselements (2) gegenüber dem Basiselement (7) in den zwei Winkelfreiheitsgraden durch Bedienung des Klemmmechanismus mittels des Bedienelements (3) durch Klemmung fixierbar ist.

3. Nadelführungsvorrichtung (1) nach einem der vorhergehenden Ansprüche, **dadurch gekennzeichnet, dass** die Winkelorientierung des Nadelführungselements (2) gegenüber dem Basiselement (7) in den zwei Winkelfreiheitsgraden stufenlos einstellbar ist.

4. Nadelführungsvorrichtung (1) nach einem der Ansprüche 1 bis 3, **dadurch gekennzeichnet, dass** eine erste Winkelskala (56) an dem Haltebügel (5) und/oder dem Klemmbügel (6) angeordnet ist und/oder eine zweite Winkelskala (89) im Bereich einer Befestigungsanordnung des Haltebügels (5) an dem Basiselement (7) angeordnet ist.

5. Nadelführungsvorrichtung (1) nach einem der vorhergehenden Ansprüche, **dadurch gekennzeichnet, dass** das Bedienelement (3) als ringförmiges Bedienelement (3) mit einem inneren Hohlraum (31) ausgebildet ist, wobei sich zumindest ein Teil des Nadelführungselements (2) durch den inneren Hohlraum (31) hindurch erstreckt.

6. Set mit mehreren Elementen für die Durchführung einer Untersuchung und/oder Behandlung eines Patienten, wobei das Set wenigstens folgende Elemente aufweist:
a) die Nadelführungsvorrichtung (1) gemäß einem der Ansprüche 1 bis 5, wobei das Basiselement (7) einen Grundkörper (70) aufweist, der eine Nadeldurchführöffnung (71) aufweist und wobei der Grundkörper (70) mehrere Positionsmarkierungen (72) aufweist, wobei mittels dieser Positionsmarkierungen (72) eine präzise Positionierung der Nadelführungsvorrichtung (1) entsprechend einem patientenbezogenen Koordinatensystem am Patienten erfolgen kann,
b) einen Rechner (100) mit einem Computerprogramm (101) oder zumindest ein solches Computerprogramm (101) für einen Rechner (100), wobei das Computerprogramm (101) dazu eingerichtet ist, anhand von Kenndaten der Nadelführungsvorrichtung (1), Untersuchungsdaten des Patienten aus einer bildgebenden Untersuchung des Patienten und wenigstens eines vorgegebenen, mit der Nadelführungsvorrichtung (1) durchzuführenden Untersuchungs- und/oder Behandlungsschritts
b1) Positionierungsangaben zur Positionierung der Nadelführungsvorrichtung (1) an dem Patienten und
b2) eine oder mehrere Winkelorientierungsangaben zur Einstellung der Winkelorientierung des Nadelführungselements (2) gegenüber dem Basiselement (7) in dem wenigstens einen Winkelfreiheitsgrad oder den mehreren voneinander unabhängigen Winkelfreiheitsgraden
zu berechnen und dem Anwender auszugeben, wenn das Computerprogramm (101) auf dem Rechner (100) ausgeführt wird.

7. Set nach Anspruch 6, **dadurch gekennzeichnet, dass** die Untersuchungsdaten des Patienten Untersuchungsdaten einer Röntgen-basierten, MRT- und/oder Ultraschall-Untersuchung umfassen.

8. Set nach einem der Ansprüche 6 oder 7, **dadurch gekennzeichnet, dass** das Set als weiteres Element eine Orientierungshilfe (200) aufweist, an der Markierungen (202) angebracht sind, die ein zweidimensionales Koordinatengitter definieren, wobei die Orientierungshilfe (200) eine Befestigungsflache aufweist, die dazu eingerichtet ist, die Orientierungshilfe (200) an dem Patienten zu befestigen.

9. Set nach Anspruch 8, **dadurch gekennzeichnet, dass** die Orientierungshilfe (200) als flaches flexibles Element ausgebildet ist.

10. Set nach einem der Ansprüche 8 oder 9, **dadurch gekennzeichnet, dass** die Untersuchungsdaten des Patienten Lageinformationen der am Patienten befestigten Orientierungshilfe (200) beinhalten, wobei das Computerprogramm (101) dazu eingerichtet ist, die Ausgabe der Positionierungsangaben und/oder der Winkelorientierungsangaben zusätzlich in Abhängigkeit von den Lageinformationen der Orientierungshilfe (200) am Patienten zu berechnen.

11. Set nach einem der Ansprüche 8 bis 10, **dadurch gekennzeichnet, dass** die Orientierungshilfe (200) mehrere Markerelemente (203) aufweist, die anhand von Röntgen-basierten, MRT- und/oder Ultraschall-Bildern automatisch detektierbar sind.

12. Computerprogramm (101) für einen Rechner (100), wobei das Computerprogramm (101) dazu eingerichtet ist, anhand von
- Kenndaten einer Nadelführungsvorrichtung (1) gemäß einem der Ansprüche 1 bis 5, wobei das Basiselement (7) einen Grundkörper (70) aufweist, der eine Nadeldurchführöffnung (71) aufweist und wobei der Grundkörper (70) mehrere Positionsmarkierungen (72) aufweist, wobei mittels dieser Positionsmarkierungen (72) eine präzise Positionierung der Nadelführungsvorrichtung (1) entsprechend einem patientenbezogenen Koordinatensystem am Patienten erfolgen kann,
- Untersuchungsdaten eines Patienten aus einer bildgebenden Untersuchung des Patienten und
- wenigstens eines vorgegebenen, mit der Nadelführungsvorrichtung (1) durchzufuhrenden Untersuchungs- und/oder Behandlungsschritts
b1) Positionierungsangaben zur Positionierung der Nadelführungsvorrichtung (1) an dem Patienten
und
b2) eine oder mehrere Winkelorientierungsangaben zur Einstellung der Winkelorientierung des Nadelführungselements (2) gegenüber dem Basiselement (7) in den zwei voneinander unabhängigen Winkelfreiheitsgraden
zu berechnen und dem Anwender auszugeben, wenn das Computerprogramm (101) auf dem Rechner (100) ausgeführt wird.

## Claims

1. Needle guiding device (1) for guiding and positioning a needle-shaped device (10) on a patient, wherein the needle guiding device (1) comprises a base element (7) and a needle guiding element (2) on which the needle-shaped device (10) can be guided along the longitudinal extent thereof, wherein the angular orientation of the needle guiding element (2) relative to the base element (7) can be set in two mutually independent degrees of angular freedom, comprising the following features:
(a) the needle guiding device (1) comprises an operating element (3) by means of which the angular orientation of the needle guiding element (2) can be fixed in two degrees of angular freedom or such fixing can be undone again,
(b) the needle guiding device (1) comprises, for each of the two settable degrees of angular freedom, an angle scale and an angle indicator (42, 55) assigned to the relevant angle scale (56, 89), such that a currently set angular orientation of the needle guiding element (2) in the two degrees of angular freedom can be read by the user based on the position of the relevant angle indicator (42, 55) relative to the assigned angle scale (56, 89),
wherein the needle guiding device (1) comprises a retaining bracket (5) to which the needle guiding element (2) is fastened, wherein the retaining bracket (5) can be moved in a first of the two degrees of angular freedom relative to the base element (7) and the needle guiding element (2) can be moved in a second of the two degrees of angular freedom relative to the retaining bracket (5), **characterised in that** the needle guiding device (1) comprises a clamping bracket (6) which is fastened to the base element (7) and can be pivoted about the same pivot axis relative to the base element (7) as the retaining bracket (5).

2. Needle guiding device (1) according to claim 1, **characterised in that** the needle guiding device (1) comprises a clamping mechanism that can be actuated by means of the operating element (3), wherein the angular orientation of the needle guiding element (2) relative to the base element (7) in the two degrees of angular freedom can be fixed by means of clamping by operating the clamping mechanism using the operating element (3).

3. Needle guiding device (1) according to any of the preceding claims, **characterised in that** the angular orientation of the needle guiding element (2) relative to the base element (7) is infinitely adjustable in the two degrees of angular freedom.

4. Needle guiding device (1) according to any of claims 1 to 3, **characterised in that** a first angle scale (56) is arranged on the retaining bracket (5) and/or clamping bracket (6) and/or a second angle scale (89) is arranged in the region of a fastening assembly of the retaining bracket (5) to the base element (7).

5. Needle guiding device (1) according to any of the preceding claims, **characterised in that** the operating element (3) is designed as an annular operating element (3) comprising an internal cavity (31), wherein at least part of the needle guiding element (2) extends through the internal cavity (31).

6. Set comprising a plurality of elements for carrying out an examination and/or treatment of a patient, wherein the set comprises at least the following elements:
a) the needle guiding device (1) according to any of claims 1 to 5, wherein the base element (7) comprises a main body (70) which comprises a needle passage opening (71) and wherein the main body (70) comprises a plurality of position markings (72), wherein precise positioning of the needle guiding device (1) on the patient in accordance with a patient-related coordinate system can take place using these position markings (72),
b) a computer (100) comprising a computer program (101) or at least a computer program (101) of this kind for a computer (100), wherein the computer program (101) is configured, on the basis of characteristic data of the needle guiding device (1), examination data relating to the patient from an imaging examination of the patient and at least one predefined examination and/or treatment step to be carried out with the needle guiding device (1), to calculate
b1) positioning information for positioning the needle guiding device (1) on the patient and
b2) one of more items of angular orientation information for setting the angular orientation of the needle guiding element (2) relative to the base element (7) in the at least one degree of angular freedom or the plurality of mutually independent degrees of angular freedom
and to output same to the user when the computer program (101) is executed on the computer (100).

7. Set according to claim 6, **characterised in that** the examination data relating to the patient include examination data from an X-ray-based, MRT and/or ultrasound examination.

8. Set according to any of claims 6 or 7, **characterised in that** the set comprises an orientation aid (200) as a further element, on which markings (202) are provided which define a two-dimensional coordinate grid, wherein the orientation aid (200) comprises a fastening surface that is designed to fasten the orientation aid (200) to the patient.

9. Set according to claim 8, **characterised in that** the orientation aid (200) is designed as a flat flexible element.

10. Set according to any of claims 8 or 9, **characterised in that** the examination data relating to the patient contain position information relating to the orientation aid (200) fastened to the patient, wherein the computer program (101) is configured to calculate the output of the positioning information and/or angular orientation information additionally depending on the position information relating to the orientation aid (200) on the patient.

11. Set according to any of claims 8 to 10, **characterised in that** the orientation aid (200) comprises a plurality of marker elements (203) which can be automatically detected based on X-ray-based, MRT or ultrasound images.

12. Computer program (101) for a computer (100), wherein the computer program (101) is configured to calculate, based on
- characteristic data of a needle guiding device (1) according to any of claims 1 to 5, wherein the base element (7) comprises a main body (70) which comprises a needle passage opening (71) and wherein the main body (70) comprises a plurality of position markings (72), wherein precise positioning of the needle guiding device (1) on the patient in accordance with a patient-related coordinate system can take place using these position markings (72),
- examination data relating to a patient from an imaging examination of the patient and
- at least one predefined examination and/or treatment step to be carried out with the needle guiding device (1),
b1) position information for positioning the needle guiding device (1) on the patient
and
b2) one of more items of angular orientation information for setting the angular orientation of the needle guiding element (2) relative to the base element (7) in the two mutually independent degrees of angular freedom
and to output same to the user when the computer program (101) is executed on the computer (100).

## Revendications

1. Dispositif de guidage d'aiguille (1) pour guider et positionner un dispositif en forme d'aiguille (10) sur un patient, le dispositif de guidage d'aiguille (1) comportant un élément de base (7) et un élément de guidage d'aiguille (2) sur lequel le dispositif en forme d'aiguille (10) peut être guidé le long de son extension longitudinale, l'orientation angulaire de l'élément de guidage d'aiguille (2) pouvant être réglée par rapport à l'élément de base (7) selon deux degrés de liberté angulaires indépendants l'un de l'autre, présentant les caractéristiques suivantes :
a) le dispositif de guidage d'aiguille (1) comporte un élément de commande (3) permettant de fixer l'orientation angulaire de l'élément de guidage d'aiguille (2) dans les deux degrés de liberté angulaires ou de défaire à nouveau une telle fixation,
b) le dispositif de guidage d'aiguille (1) comporte, pour chacun des deux degrés de liberté angulaires réglables, une échelle angulaire et un indicateur angulaire (42, 55) associé à l'échelle angulaire respective (56, 89), de sorte que l'utilisateur peut lire une orientation angulaire actuellement réglée de l'élément de guidage d'aiguille (2) dans les deux degrés de liberté angulaires à l'aide de la position de l'indicateur angulaire respectif (42, 55) par rapport à l'échelle angulaire associée (56, 89),
le dispositif de guidage d'aiguille (1) comportant un étrier de retenue (5) auquel est fixé l'élément de guidage d'aiguille (2), l'étrier de retenue (5) étant mobile dans un premier des deux degrés de liberté angulaires par rapport à l'élément de base (7) et l'élément de guidage d'aiguille (2) étant mobile dans un deuxième des deux degrés de liberté angulaire par rapport à l'étrier de retenue (5), **caractérisé en ce que** le dispositif de guidage d'aiguille (1) comporte un étrier de serrage (6) fixé à l'élément de base (7) et pivotable par rapport à l'élément de base (7) autour du même axe de pivotement que l'étrier de retenue (5).

2. Dispositif de guidage d'aiguille (1) selon la revendication 1, **caractérisé en ce que** le dispositif de guidage d'aiguille (1) comporte un mécanisme de serrage actionnable par l'élément de commande (3), l'orientation angulaire de l'élément de guidage d'aiguille (2) par rapport à l'élément de base (7) dans les deux degrés de liberté angulaire pouvant être fixé par serrage en actionnant le mécanisme de serrage au moyen de l'élément de commande (3).

3. Dispositif de guidage d'aiguille (1) selon l'une des revendications précédentes, **caractérisé en ce que** l'orientation angulaire de l'élément de guidage d'aiguille (2) par rapport à l'élément de base (7) est réglable en continu dans les deux degrés de liberté angulaires.

4. Dispositif de guidage d'aiguille (1) selon l'une des revendications 1 à 3, **caractérisé en ce qu'**une première échelle angulaire (56) est disposée sur l'étrier de retenue (5) et/ou l'étrier de serrage (6) et/ou une deuxième échelle angulaire (89) est disposée sur l'élément de base (7) dans la zone d'un dispositif de fixation de l'étrier de retenue (5).

5. Dispositif de guidage d'aiguille (1) selon l'une des revendications précédentes, **caractérisé en ce que** l'élément de commande (3) est conçu comme un élément de commande annulaire (3) avec une cavité intérieure (31), au moins une partie de l'élément de guidage d'aiguille (2) s'étendant à travers la cavité intérieure (31).

6. Ensemble comprenant plusieurs éléments destinés à la réalisation d'un examen et/ou d'un traitement d'un patient, l'ensemble comprenant au moins les éléments suivants :
a) le dispositif de guidage d'aiguille (1) selon l'une des revendications 1 à 5, dans lequel l'élément de base (7) comporte un corps de base (70) qui présente une ouverture de guidage d'aiguille (71) et dans lequel le corps de base (70) comporte plusieurs repères de position (72), ces repères de position (72) permettant un positionnement précis du dispositif de guidage d'aiguille (1) sur le patient selon un système de coordonnées propre au patient,
b) un ordinateur (100) avec un programme informatique (101) ou au moins un tel programme informatique (101) pour un ordinateur (100), le programme informatique (101) étant conçu pour, à partir des données caractéristiques du dispositif de guidage d'aiguille (1), calculer des données d'examen du patient issues d'un examen d'imagerie du patient et d'au moins une étape d'examen et/ou de traitement prédéfinie à réaliser avec le dispositif de guidage d'aiguille (1)
b1) calculer des indications de positionnement pour positionner le dispositif de guidage d'aiguille (1) sur le patient et
b2) calculer une ou plusieurs indications d'orientation angulaire pour régler l'orientation angulaire de l'élément de guidage d'aiguille (2) par rapport à l'élément de base (7) dans au moins un degré de liberté angulaire ou dans plusieurs degrés de liberté angulaire indépendants les uns des autres,
et les transmettre à l'utilisateur lorsque le programme informatique (101) est exécuté sur l'ordinateur (100).

7. Ensemble selon la revendication 6, **caractérisé en ce que** les données d'examen du patient comprennent des données d'examen issues d'un examen radiographique, IRM et/ou échographique.

8. Ensemble selon l'une des revendications 6 ou 7, **caractérisé en ce que** l'ensemble comprend comme élément supplémentaire un guide d'orientation (200) sur lequel sont apposés des repères (202) qui définissent une grille de coordonnées bidimensionnelle, le guide d'orientation (200) comportant une surface de fixation conçue pour
fixer le guide d'orientation (200) sur le patient.

9. Ensemble selon la revendication 8, **caractérisé en ce que** le guide d'orientation (200) est conçu comme un élément plat et flexible.

10. Ensemble selon l'une des revendications 8 ou 9, **caractérisé en ce que** les données d'examen du patient contiennent des informations de position de l'aide à l'orientation (200) fixée sur le patient, le programme informatique (101) étant conçu pour calculer en outre la sortie des indications de positionnement et/ou des indications d'orientation angulaire en fonction des informations de position du guide d'orientation (200) sur le patient.

11. Ensemble selon l'une des revendications 8 à 10, **caractérisé en ce que** le guide d'orientation (200) comporte plusieurs éléments marqueurs (203) qui peuvent être détectés automatiquement à l'aide d'images radiographiques, IRM et/ou échographiques.

12. Programme informatique (101) pour un ordinateur (100), le programme informatique (101) étant conçu pour calculer,
- à l'aide de données caractéristiques d'un dispositif de guidage d'aiguille (1) selon l'une des revendications 1 à 5, dans lequel l'élément de base (7) comporte un corps de base (70) qui présente une ouverture de guidage d'aiguille (71) et dans lequel le corps de base (70) comporte plusieurs repères de position (72), ces repères de position (72) permettant un positionnement précis du dispositif de guidage d'aiguille (1) sur le patient selon un système de coordonnées propre au patient,
- des données d'examen d'un patient issues d'un examen d'imagerie du patient et
- d'au moins une étape d'examen et/ou de traitement prédéfinie à réaliser avec le dispositif de guidage d'aiguille (1)
b1) des indications de positionnement pour positionner le dispositif de guidage d'aiguille (1) sur le patient et
b2) une ou plusieurs indications d'orientation angulaire pour régler l'orientation angulaire de l'élément de guidage d'aiguille (2) par rapport à l'élément de base (7) dans les deux degrés de liberté angulaires indépendants l'un de l'autre
et les transmettre à l'utilisateur lorsque le programme informatique (101) est exécuté sur l'ordinateur (100).
